# EUROPÄISCHE PATENTSCHRIFT

(11) **EP 1 827 333 B1**
(45) Veröffentlichungstag und Bekanntmachung des Hinweises auf die Patenterteilung: **20.10.2010**
(21) Anmeldenummer: 04803811.1
(22) Anmeldetag: 13.12.2004
(51) Int. Cl.: A61F 13/04, D03D 19/00, D03D 1/00

(54) **MATERIAL ZUR HERSTELLUNG EINES STÜTZVERBANDES**
MATERIAL FOR PRODUCING A SUPPORT BANDAGE
MATERIAU SERVANT A PRODUIRE UNE BANDE DE MAINTIEN

(43) Veröffentlichungstag der Anmeldung: 05.09.2007
(60) Teilanmeldung: 09004477.7
(73) Patentinhaber: Lohmann & Rauscher GmbH & Co. KG, 56567 Neuwied (DE)
(72) Erfinder: LEUPRECHT, Helmut, A-1130 Wien (AT)
(74) Vertreter: Leinweber & Zimmermann
(86) Internationale Anmeldenummer: PCT/EP2004/014181
(87) Internationale Veröffentlichungsnummer: WO 2006/063599

(56) Entgegenhaltungen:
- DE-U1- 20 011 824
- GB-A- 928 099
- US-A- 4 207 885

## Beschreibung

Die Erfindung betrifft ein Material zur Herstellung eines Stützverbandes mit einem mit einem härtbaren Kunststoff beschichteten und/oder imprägnierten und zumindest teilweise in Form eines zwei sich zwischen nebeneinander verlaufenden Schußfäden kreuzende Kettfäden aufweisenden Drehergewebes gebildeten textilen Träger.

Materialien mit einem mit einem härtbaren Kunststoff beschichteten und/oder imprägnierten textilen Träger werden als Ersatz für Gipsverbände eingesetzt, welche insbesondere aufgrund ihres hohen Gewichtes einen schlechten Tragekomfort bieten. Dagegen können die genannten Ersatzmaterialien in Form von Kunststoff-Festverbänden durch Wahl entsprechender Kunststoffe und Träger bei einem wesentlich geringeren Gewicht mit der gleichen Festigkeit bzw. Steifigkeit hergestellt werden, während gleichzeitig eine hinreichende Atmungsaktivität sichergestellt ist. Darüber hinaus können derartige Materialien auch zur Herstellung eines auch in ausgehärtetem Zustand noch geringfügig verformbaren Verbandes eingesetzt werden, wenn härtbare Kunststoffe mit einer entsprechenden Beschaffenheit eingesetzt werden.

Herkömmliche Kunststoff-Festverbände werden in Form eines bahnförmigem, bandagenartigen Materials geliefert und zur Herstellung des Stützverbandes um den zu stützenden Körperteil gewickelt, wobei die Aushärtung des Kunststoffes durch eine entsprechende Behandlung vor, während und/oder nach Anlegen des Verbandes veranlaßt wird. Je nach Kunststoff kann dabei eine UV-Härtung, eine Wärmehärtung oder eine Härtung mit Hilfe eines Lösungsmittels durchgeführt werden. Als besonders zweckmäßig hat sich dabei die Härtung mit Hilfe von kaltem Wasser als Lösungsmittel erwiesen, weil dadurch keine zu Hautirritationen führende Erwärmung des Kunststoffs und auch sonst keine Hautreaktion hervorgerufen wird.

Zur Anpassung des herkömmlichen Materials an die Körperform können in Längs- und Querrichtung dehnbare textile Träger eingesetzt werden, wobei die während des Anlegens des Verbandes erzeugte Verformung des Trägers durch die Aushärtung des Kunststoffes fixiert wird. Bei herkömmlichen Kunststoff-Festverbänden werden zum Erhalt der gewünschten Dehnbarkeit gewirkte Glasfaserträger eingesetzt, welche ihre Dehnbarkeit in Querrichtung, d. h. in Schußrichtung des Trägers, aus der Maschenstruktur des gewirkten Materials beziehen und deren Dehnbarkeit in Längsrichtung des bahnförmigen, bandagenartigen Materials, d. h. in Kettrichtung des gewirkten Trägers, auf die Steifigkeit der Glasfasern zurückzuführen ist, welche dazu führt, daß die einzelnen Maschen des gewirkten Trägers auch in der Kettrichtung noch verformbar bleiben.

Unter Verwendung derartiger textiler Träger hergestellte Materialien der vorstehend angegebenen Art sind beispielsweise in der US-PS-3,787,272 angegeben.

Beim Einsatz dieser Materialien wird es jedoch als problematisch angesehen, daß beim Zerschneiden daraus hergestellter Stützverbände Glasfaserstäube entstehen können, welche im Verdacht stehen, gesundheitsschädlich zu sein.

Angesichts dieser Probleme wurde bereits die Herstellung textiler Träger aus anderen Kunstfasern, wie etwa Polyestergarnen und Polyamidgarnen, vorgeschlagen. Diese Garne weisen jedoch eine wesentlich geringere Steifigkeit auf als Glasfasern, so daß unter Verwendung derartiger Garne hergestellte Materialien zur Herstellung von Stützverbänden üblicherweise nur in der Querrichtung, d.h. der Schußrichtung des gewirkten textilen Trägers, eine ausreichende Dehnbarkeit aufweisen, während sie in der Längsrichtung bzw. Kettrichtung nur noch eine geringfügige Verformbarkeit zeigen. Daher passen sich unter Verwendung derartiger Träger hergestellte Materialien nur schlecht an die Körperform an. Zur Lösung dieses Problems wird in der EP 0 356 446 B1 ein gewirkter Träger für ein Material zur Herstellung eines Stützverbandes vorgeschlagen, bei dem die in Längsrichtung verlaufenden Kettfäden aus einem wärmeschrumpfbaren Material bestehen. Der in dieser Schrift beschriebene Träger wird zunächst aus im allgemeinen als Multifilamentgarne vorliegenden Garnen gewirkt und dann einer Wärmebehandlung unterzogen. Diese Wärmebehandlung führt zu einer Schrumpfung der in Längsrichtung des Trägers verlaufenden Kettfäden, welche diesen Kettfäden und damit auch dem Träger als Ganzem eine Strukturveränderung verleiht, die wiederum eine Dehnung der Kettfäden und damit auch des gesamten Trägers in Längsrichtung ermöglicht.

Wenngleich mit derartigen Trägern eine zufriedenstellende Anpassung des daraus hergestellten Verbandmaterials an die Körperform erreicht werden kann, ist die Herstellung dieser Träger problematisch.

Angesichts dieser Probleme im Stand der Technik wurde in der DE 200 11 824 vorgeschlagen, einen Stützverbandträger der eingangs beschriebenen Art einzusetzen. Dabei können durch Einsatz eines im Vergleich zu gewirkten Strukturen einfachen Drehergewebes die angesprochenen Herstellungsprobleme gelöst und gleichzeitig eine zufriedenstellende Formstabilität des Trägermaterials erreicht werden. Dabei wird die Herstellung der bekannten Materialien mit einem Träger in Form eines Drehergewebes dadurch vereinfacht, daß einer der sich kreuzenden Kettfäden unterhalb der Schußfäden verläuft und der andere der sich kreuzenden Kettfäden oberhalb der Schußfäden angeordnet ist, wobei der unterhalb der Schußfäden verlaufende Kettfaden an den Kreuzungsstellen oberhalb des oberhalb der Schußfäden verlaufenden Kettfadens angeordnet ist.

Wenngleich derartige Materialien einfach herstellbar sind, eine zufriedenstellende Formstabilität gewährleisten und damit auch die zur Anpassung an die Körperform gewünschte Dehnbarkeit sichergestellt werden kann, hat es sich gezeigt, daß die Langzeitstabilität dieser Materialien noch zu wünschen übrig läßt.

Angesichts dieser Probleme im Stand der Technik liegt der Erfindung die Aufgabe zugrunde, eine Weiterbildung der bekannten Materialien anzugeben, welche auch bei längerem Einsatz stabil bleibt.

Erfindungsgemäß wird diese Aufgabe durch eine Weiterbildung des bekannten Materials gelöst, die im wesentlichen dadurch gekennzeichnet ist, daß das Drehergewebe mindestens einen zwischen zwei Paaren sich kreuzender Kettfäden angeordneten und keinen anderen Kettfaden kreuzenden zusätzlichen Kettfaden aufweist.

Diese Erfindung geht auf die Erkenntnis zurück, daß durch die gute Fixierung der Schußfäden mit Hilfe der sich bei einem Drehergewebe kreuzenden Kettfäden zwar eine im Vergleich zu herkömmlichen Geweben besonders gute Formstabilität erreichbar ist, jedoch die strukturelle Integrität des Materials in Dickenrichtung, d. h. in einer senkrecht zu einer durch Kettfäden und Schußfäden aufgespannten Ebene, zu wünschen übrig läßt, weil einzelne Kettfäden eines herkömmlichen Drehergewebes immer nur auf einer Seite der Schußfäden verlaufen. Das führt zu einer mangelhaften Haftung des auf den Träger aufgebrachten Kunststoffs und darüber hinaus auch noch dazu, daß einzelne Kettfäden an Rändern des Materials herausrutschen können. Durch diese Effekte werden die beobachteten Probleme hinsichtlich der Langzeitstabilität herkömmlicher Materialien der eingangs beschriebenen Art verursacht. Bei erfindungsgemäß weitergebildeten Materialien wird diesen Effekten durch Einsatz der zusätzlichen Kettfäden entgegengewirkt, welche einerseits eine strukturelle Integrität des Materials in Dickenrichtung bewirken und andererseits eine gute Fixierung nicht nur der Schußfäden, sondern auch der Kettfäden des Materials gewährleisten.

Herstellungstechnisch hat es sich als besonders günstig erwiesen, wenn mindestens ein zusätzlicher Kettfaden mindestens einen auf einer einem ersten Abschnitt dieses Kettfadens abgewandten Seite der Schußfäden verlaufenden zweiten Abschnitt aufweist, wobei der Übergang zwischen dem ersten Abschnitt und dem zweiten Abschnitt vorzugsweise zwischen denjenigen nebeneinander verlaufenden Schußfäden erfolgt, zwischen denen sich auch die Kettfäden mindestens eines Paares sich kreuzender Kettfäden kreuzen. Dabei kann die strukturelle Integrität des Materials in Dickenrichtung weitergefördert werden, wenn von zwei auf einander entgegengesetzten Seiten eines Paares sich kreuzender Kettfäden angeordneten zusätzlichen Kettfäden ein zusätzlicher Kettfaden im Bereich der Schußfäden auf der dem anderen zusätzlichen Kettfaden abgewandten Seite der Schußfäden angeordnet ist. Im Hinblick auf die angestrebte Verhinderung des Herausrutschens einzelner Kettfäden an einem Rand des Materials, welches ein Ausfransen des Materials an diesem Rand verursachen kann, hat es sich als besonders günstig erwiesen, wenn mindestens ein zusätzlicher Kettfaden und/oder mindestens ein Schußfaden texturiert ist.

Wie der vorstehenden Erläuterung bekannter Materialien zu entnehmen ist, ist bei dem erfindungsgemäßen Material vorzugsweise mindestens ein Kettfaden eines Paars sich kreuzender Kettfäden und/oder mindestens ein zusätzlicher Kettfaden und/oder mindestens ein Schußfaden dehnbar, vorzugsweise elastisch dehnbar, und besteht insbesondere zweckmäßigerweise aus einem elastisch dehnbaren Material, wie etwa elastischem Polyurethan. Dabei kann der zumindest teilweise aus elastischem Material bestehende Kettfaden eine vorzugsweise zweifach mit einem weiteren Garn, wie etwa einem Polyestergarn, umwickelte Seele aus elastischem Garn, wie etwa Polyurethangarn, aufweisen.

Bei Einsatz herkömmlicher Materialien der eingangs beschriebenen Art wird in einigen Fällen eine Einschnürung unter Zugbelastung und/oder die Ausbildung wulstartiger Falten beobachtet. Dabei können sich diese beobachteten wulstartigen Falten bei einem Unterschenkelverband ringförmig um die Fessel und auf dem Vorfuß ausbilden. Die Falten laufen dabei an den genannten Stellen parallel zur Kettrichtung des Trägermaterials.

Bei einem erfindungsgemäßen Material können diese Probleme gelöst werden, wenn mindestens einen Schußfaden des Drehergewebes zumindest abschnittweise aus einem hochfesten Garn, wie etwa einem Polyestergarn, auszuführen.

Diese Lösung geht auf die Erkenntnis zurück, daß die beobachtete Ausbildung von Einschnürungen und wulstartigen Falten auf eine zu geringe Querstabilität des herkömmlichen Trägermaterials hervorgerufen wird. Dieser Mangel kann gemäß dem gerade erläuterten Gesichtspunkt der Erfindung durch den Einsatz eines hochfesten Schußfadens behoben werden, weil dieser hochfeste Schußfaden, beispielsweise in Form eines Polyestergams, dem Trägermaterial eine erhöhte Quersteifigkeit verleiht.

Zum Erhalt der gewünschten Dehnbarkeit können wärmeschrumpfbare Kettfäden und/oder Schußfäden eingesetzt werden, welche vor und/oder nach dem Webvorgang einer Wärmebehandlung unterzogen werden, um so eine dehnbare Struktur zu erhalten. Alternativ oder zusätzlich kann das Gewebe des erfindungsgemäßen Materials aber auch Kettfäden und/oder Schußfäden aus einem Elastomermaterial aufweisen, welche ihre Dehnbarkeit aus den Eigenschaften des Materials selbst und nicht aus der Fadenstruktur beziehen.

Im Hinblick auf eine ausreichende Stabilität des Materials unter gleichzeitiger Sicherstellung einer zufriedenstellenden Anpassung an die Körperform hat es sich als besonders günstig erwiesen, wenn das Material in Richtung der Kettfäden des Gewebes vor dem Härten des Kunststoffes eine Dehnbarkeit von mindestens 20 %, vorzugsweise mindestens 30 %, besonders bevorzugt etwa 60 bis 95 % aufweist, wobei das Material in Richtung der Kettfäden des Gewebes in ungedehntem Zustand zweckmäßigerweise eine Reißfestigkeit von mindestens 10 N/cm, vorzugsweise mindestens 20 N/cm, besonders bevorzugt etwa 30 bis 60 N/cm aufweist.

Unter den gleichen Gesichtspunkten (Anpassung an die Körperform und Materialstabilität) hat es sich als günstig erwiesen, wenn das Material in Richtung der Schußfäden des Gewebes vor dem Härten des Kunststoffes eine Dehnbarkeit von mindestens 30 %, vorzugsweise mindestens 40 %, besonders bevorzugt etwa 50 bis 100 % aufweist, wobei die Reißfestigkeit des Materials in Richtung der Schußfäden des Gewebes in ungedehntem Zustand zweckmäßigerweise 10 N/cm, vorzugsweise mindestens 20 N/cm, besonders bevorzugt etwa 30 bis 50 N/cm beträgt.

Ein vergleichsweise geringes Gewicht erfindungsgemäßer Materialien kann bei gleichzeitiger Sicherstellung einer ausreichend hohen Stabilität erhalten werden, wenn das Gewebe in ungedehntem Zustand ein Flächengewicht von etwa 50 bis 250 g/m², vorzugsweise etwa 100 bis 200 g/m², aufweist.

Die Schuß- und/oder Kettfäden des Gewebes des erfindungsgemäßen Materials können beispielsweise Naturfasern aufweisen. Besonders gute Eigenschaften werden jedoch erhalten, wenn die Schuß- und/oder Kettfäden des Gewebes zusätzlich oder alternativ Kunstfasern, wie etwa Polyamidfasern, Polyesterfasern, Polypropylenfasern od. dgl., aufweisen, weil derartige Kunstfasern eine besonders gute Einstellung der Dehnbarkeit, Reißfestigkeit und anderer mechanischer Eigenschaften ermöglichen. Dazu können die Schuß- und/oder Kettfäden zum Erhalt der Dehnbarkeit zumindest teilweise texturiert sein. Diese Texturierung kann durch Wärmebehandlung der Fasern oder durch Behandlung der Fasern mit einem Lösungsmittel erhalten werden.

Wie bei den bekannten Materialien gemäß DE 200 11 824, können die Drehergewebe erfindungsgemäßer Materialien unter Sicherstellung einer besonders hohen Stabilität besonders einfach hergestellt werden, wenn bei mindestens einem Paar sich kreuzender Kettfäden einer der sich kreuzenden Kettfäden unterhalb der Schußfäden verläuft, der andere der sich kreuzenden Kettfäden oberhalb der Schußfäden verläuft und der unterhalb der Schußfäden verlaufende Kettfaden an den Kreuzungsstellen oberhalb des oberhalb der Schußfäden verlaufenden Kettfadens angeordnet ist. Weiter wird eine besonders hohe Stabilität derartiger Träger in Form eines Drehergewebes erreicht, wenn mindestens zwei Schußfäden zwischen aufeinanderfolgenden Kreuzungsstellen der Kettfäden und/oder aufeinanderfolgenden Übergängen mindestens eines zusätzlichen Kettfadens angeordnet sind. In diesem Zusammenhang hat es sich im Hinblick auf den Erhalt der gewünschten erhöhten Quersteifigkeit unter gleichzeitiger Sicherstellung der ebenfalls gewünschten strukturellen Integrität als besonders günstig erwiesen, wenn mindestens einer der zwischen aufeinanderfolgenden Kreuzungsstellen und/oder aufeinanderfolgenden Übergängen angeordneten Schußfäden texturiert ist und ein anderer dieser Schußfäden aus einem hochfesten, vorzugsweisen glatten Garn, insbesondere Polyestergarn, gebildet ist. Dabei wird mit dem hochfesten Garn die gewünschte Quersteifigkeit sichergestellt, während mit dem texturierten Schußfaden die gewünschte strukturelle Integrität verbessert wird.

In dieser Beschreibung wird jedes im wesentlichen geradlinig und senkrecht zu den Kettfäden verlaufendes Fadensegment als "Schußfaden" bezeichnet. Zum Erhalt einer besonders guten Stabilität des Trägergewebes des erfindungsgemäßen Materials hat es sich als günstig erwiesen, wenn die einzelnen Schußfäden in Form von Fadensegmenten gebildet sind, von denen mindestens zwei über ein am Rand des Gewebes angeordnetes Verbindungssegment miteinander verbunden sind, wobei dieses Verbindungssegment einstückig mit den die Schußfäden bildenden Fadensegmenten vorliegen kann. Dabei kann die Bildung von Rissen oder andersartiger Beschädigungen am Rand des Gewebes vermieden werden, wenn mindestens ein zwei Schußfäden miteinander verbindendes Verbindungssegment mindestens einen, vorzugsweise mindestens zwei, vorhergehende Schußfäden umläuft, um so eine Kante des Gewebes aus miteinander verbundenen Fasersegmenten zu bilden.

Im Hinblick auf den Erhalt einer möglichst hohen Stabilität und Dehnbarkeit eines erfindungsgemäßen Materials bei gleichzeitiger Sicherstellung eines möglichst geringen Gewichtes hat es sich als günstig erwiesen, wenn das Gewebe in ungedehntem Zustand etwa 40 bis 80, vorzugsweise etwa 66 Kettfäden, d. h. bei Verwendung eines Drehergewebes etwa 20 bis 40, vorzugsweise etwa 33 Doppelfäden in Kettrichtung und/oder etwa 80 bis 150, vorzugsweise etwa 132 Schußfäden, d.h. 40 bis 75, vorzugsweise etwa 66 Doppelschußfäden bei Verwendung des vorstehend beschriebenen Drehergewebes auf 10 cm der Gewebelänge bzw. -breite aufweist.

Wie eingangs bereits erläutert, kann es sich bei dem härtbaren Kunststoff um einen wärmehärtbaren oder UV-härtbaren Kunststoff handeln. Allerdings wird auch bei der Herstellung eines erfindungsgemäßen Materials vorzugsweise ein mit einem Lösungsmittel, insbesondere kaltem Wasser, härtbarer Kunststoff eingesetzt. Im Hinblick auf den Erhalt einer möglichst hohen Hautverträglichkeit bei gleichzeitiger Sicherstellung der gewünschten mechanischen Eigenschaften hat sich die Verwendung eines Kunststoffes, der ein feuchtigkeitshärtendes Polyurethanprepolymer umfaßt, als besonders günstig erwiesen. Derartige Kunststoffe sind beispielsweise in der EP 0 093 780 B1 beschrieben, deren Offenbarungsgehalt hinsichtlich der darin beschriebenen aushärtbaren Kunststoffe hiermit durch ausdrückliche Inbezugnahme in diese Beschreibung aufgenommen wird. Zweckmäßigerweise beträgt der Anteil des Kunststoffes an dem erfindungsgemäßen Material etwa 30 bis 70 Gew.-%.

Wie der vorstehenden Erläuterung des erfindungsgemäßen Materials bereits zu entnehmen ist, zeichnet sich der zur Herstellung dieses Materials eingesetzte erfindungsgemäße Träger im wesentlichen dadurch aus, daß er eine gewebte Struktur aufweist, wobei der Träger in Form eines Drehergewebes mit zusätzlichen, keine anderen Kettfäden kreuzenden Kettfäden verwirklicht ist. Mit Hilfe eines derartigen Trägers kann das erfindungsgemäße Material mit einem erfindungsgemäßen Verfahren hergestellt werden, indem der Träger mit einem härtbaren Kunststoff beschichtet und/oder imprägniert wird, wobei der Träger vor und/oder nach der Beschichtung bzw. Imprägnierung mit dem Kunststoff einer Wärmebehandlung unterzogen werden kann, um dem Material so insgesamt die gewünschte Dehnbarkeit zu verleihen.

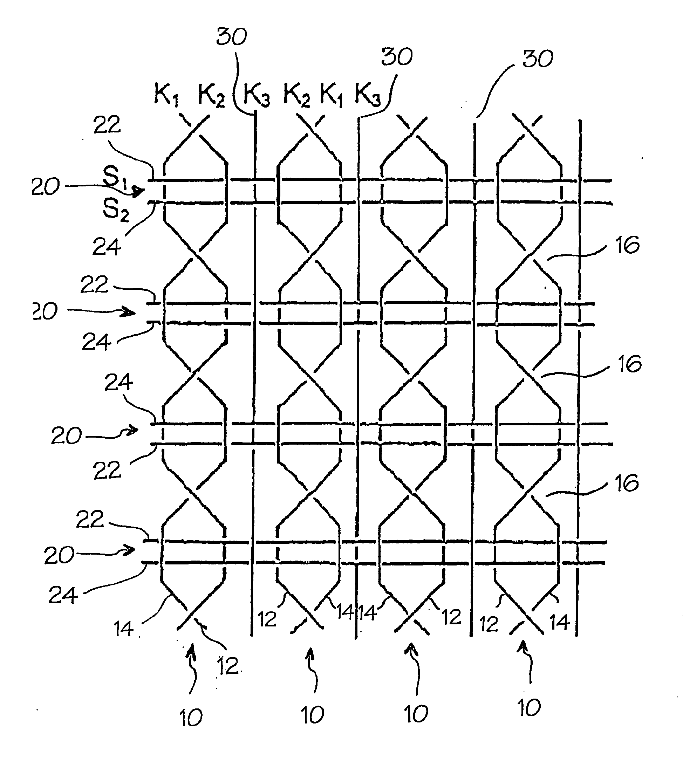

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. Die einzige Figur der Zeichnung zeigt eine schematische Darstellung einer Ausführungsform eines zur Herstellung eines erfindungsgemäßen Materials einsetzbaren erfindungsgemäßen Trägers.

Die Figur zeigt einen Ausschnitt eines erfindungsgemäßen Trägers für ein Material zur Herstellung eines Stützverbandes in Form eines Drehergewebes mit vier Kettfadenpaaren 10, mit sich kreuzenden Kettfäden 12 und 14 insgesamt vier Schußfadenpaaren 20 und drei zusätzlichen Schußfäden 30. Jedes der Kettfadenpaare 10 umfaßt einen unterhalb der Schußfadenpaare 20 verlaufenden Kettfaden 12 sowie einen oberhalb der Schußfadenpaare 20 verlaufenden Kettfaden 14. Dabei kreuzt der unterhalb der Schußfadenpaare 20 verlaufende Kettfaden 12 den oberhalb der Schußfadenpaare 20 verlaufenden Kettfaden 14 desselben Kettfadenpaares 10 an Kreuzungsstellen 16. An diesen Kreuzungsstellen 16 ist der unterhalb der Schußfadenpaare 20 verlaufende Kettfaden 12 oberhalb des oberhalb der Schußfadenpaare 20 verlaufenden Kettfadens 14 angeordnet. Zwischen aufeinanderfolgenden Kreuzungsstellen 16 der Kettfadenpaare 10 ist jeweils ein Schußfadenpaar 20 angeordnet, wobei die Kreuzungsstellen der einzelnen Kettfadenpaare 10 etwa auf einer parallel zu den Schußfadenpaaren 20 verlaufenden Geraden liegen. Durch die in der Zeichnung dargestellte Struktur eines Drehergewebes werden die Schußfadenpaare 20 fixiert, wodurch ein Verrutschen der einzelnen Fäden während der Beschichtung bzw. Imprägnierung mit dem härtbaren Kunststoff, wie etwa einem Polyurethanprepolymer, verhindert wird.

Jedes der Schußfadenpaare 20 enthält einen glatten Schußfaden 22 aus einem hochfesten Polyestergarn und einen texturierten Schußfaden 24, der ebenfalls aus einem Polyestergarn gebildet ist.

Zwischen den Kettfadenpaaren 10 sind zusätzliche, keine anderen Kettfäden kreuzende Kettfäden 30 angeordnet, welche abschnittweise oberhalb der Schußfäden und abschnittweise unterhalb der Schußfäden verlaufen. Dabei erfolgt der Übergang zwischen den oberhalb der Schußfäden verlaufenden Abschnitten und den unterhalb der Schußfäden verlaufenden Abschnitten zwischen denjenigen Schußfäden, zwischen denen sich auch die Kettfäden 12 und 14 der Kettfadenpaare 10 kreuzen. In der Zeichnung ist erkennbar, daß von zwei auf einander entgegengesetzten Seiten eines Paars sich kreuzender Kettfäden 12 und 14 angeordneten zusätzlichen Kettfäden 30 ein zusätzlicher Kettfaden 30 im Bereich der Schußfäden auf der dem anderen zusätzlichen Kettfaden 30 abgewandten Seite dieser Schußfäden 22, 24 angeordnet ist.

Die Erfindung ist nicht auf den Einsatz der anhand der Zeichnung erläuterten Drehergewebe beschränkt. Vielmehr können zur Herstellung eines erfindungsgemäßen Trägers auch Fäden aus natürlichen Fasern, Polyamidfasern, Polypropylenfasern u. dgl. eingesetzt werden. Dabei kann die Dehnbarkeit des unter Verwendung von Polyamidfasern hergestellten Trägers in ausgerüstetem, d. h. mit dem Kunststoff versehenem, ungedehntem Zustand in Kettrichtung 80 bis 90 % betragen. Das bedeutet, daß das ungedehnte Gewebe in Kettrichtung von 100 auf 180 bis 190 % gedehnt werden kann. In Schußrichtung kann die Dehnbarkeit eines aus Polyamidfasern hergestellten Trägers in ausgerüstetem (ungedehntem) Zustand 120 bis 130 % betragen. Das heißt, daß dieses Gewebe von 100 auf 220 bis 230 % gedehnt werden kann. Dabei kann das Flächengewicht in gedehntem Zustand 50 bis 55 g/m² betragen. Bei Einsatz von Polyamidfasern zur Herstellung der Kett- und Schußfäden kann eine Kettfadenstärke im Bereich von 40 bis 60 tex, vorzugsweise etwa 46,8 tex, und eine Schußfadenstärke im Bereich von 20 bis 40, vorzugsweise etwa 31,2 tex, zum Einsatz kommen.

Ferner können im Rahmen der Erfindung auch neue Kunststoffe, insbesondere an den Einsatz mit Polyamid angepaßte Kunststoffe in Form eines feuchtigkeitshärtenden Polyurethanprepolymers zum Einsatz kommen.

## Patentansprüche

1. Material zur Herstellung eines Stützverbandes mit einem mit einem härtbaren Kunststoff beschichteten und/oder imprägnierten und zumindest teilweise in Form eines zwei sich zwischen nebeneinander verlaufenden Schußfäden (22, 24) kreuzende Kettfäden (12, 14) aufweisenden Drehergewebes gebildeten Träger, **dadurch gekennzeichnet, daß** das Drehergewebe mindestens einen zwischen zwei Paaren (10) sich kreuzender Kettfäden (12, 14) angeordneten und keinen anderen Kettfaden kreuzenden zusätzlichen Kettfaden (30) aufweist.

2. Material nach Anspruch 1, **dadurch gekennzeichnet, daß** mindestens ein zusätzlicher Kettfaden (30) mindestens einen auf einer einem ersten Abschnitt dieses Kettfadens (30) abgewandten Seite der Schußfäden (22, 24) verlaufenden zweiten Abschnitt aufweist, wobei der Übergang zwischen dem ersten Abschnitt und dem zweiten Abschnitt vorzugsweise zwischen denjenigen nebeneinander verlaufenden Schußfäden (22, 24) erfolgt, zwischen denen sich auch die Kettfäden (12, 14) mindestens eines Paares (10) sich kreuzender Kettfäden (12, 14) kreuzen.

3. Material nach Anspruch 1 oder 2, **dadurch gekennzeichnet, daß** von zwei auf einander entgegengesetzten Seiten eines Paares (10) sich kreuzender Kettfäden (12, 14) angeordneten zusätzlichen Kettfäden (30) ein zusätzlicher Kettfaden (30) im Bereich der Schußfäden (22, 24) auf der dem anderen zusätzlichen Kettfaden (30) abgewandten Seite der Schußfäden (22, 24) angeordnet ist.

4. Material nach einem der Ansprüche 1 bis 3, **dadurch gekennzeichnet, daß** mindestens ein zusätzlicher Kettfaden (30) texturiert ist.

5. Material nach einem der vorhergehenden Ansprüche, **dadurch gekennzeichnet, daß** mindestens ein Kettfaden (12, 14) eines Paares (10) sich kreuzender Kettfäden (12, 14) und/oder mindestens ein zusätzlicher Kettfaden (30) und/oder mindestens ein Schußfaden (22, 24) dehnbar ist, vorzugsweise elastisch dehnbar ist, insbesondere zumindest teilweise aus einem elastisch dehnbaren Material, wie etwa elastischem Polyurethan, besteht.

6. Material nach Anspruch 5, **dadurch gekennzeichnet, daß** der zumindest teilweise aus elastischem Material bestehende Kettfaden (14) eine vorzugsweise zweifach mit einem weiteren Garn, wie etwa einem Polyestergarn, umwickelte Seele aus elastischem Garn, wie etwa Polyurethangarn, aufweist.

7. Material nach einem der vorhergehenden Ansprüche, **dadurch gekennzeichnet, dass** das Drehergewebe mindestens einen ein hochfestes Garn, insbesondere Polyestergarn, aufweisenden Schussfaden (22) aufweist.

8. Material nach einem der vorhergehenden Ansprüche, **dadurch gekennzeichnet, daß** das Gewebe wärmeschrumpfbare Kettfäden und/oder Schußfäden aufweist.

9. Material nach einem der vorhergehenden Ansprüche, **dadurch gekennzeichnet, daß** das Gewebe Kettfäden und/oder Schußfäden aus einem Elastomermaterial aufweist.

10. Material nach einem der vorhergehenden Ansprüche, **dadurch gekennzeichnet, daß** das Material in Richtung der Kettfäden (22, 24, 30) des Gewebes vor dem Härten des Kunststoffes eine Dehnbarkeit von mindestens 20 %, vorzugsweise mindestens 30 %, besonders bevorzugt etwa 65 bis 95 % aufweist.

11. Material nach einem der vorhergehenden Ansprüche, **dadurch gekennzeichnet, daß** das Material in Richtung der Kettfäden (22, 24, 30) des Gewebes in ungedehntem Zustand eine Reißfestigkeit von mindestens 10 N/cm, vorzugsweise mindestens 20 N/cm, besonders bevorzugt etwa 30 bis 60 N/cm aufweist.

12. Material nach einem der vorhergehenden Ansprüche, **dadurch gekennzeichnet, daß** das Material in Richtung der Schußfäden (22, 24) des Gewebes vor dem Härten des Kunststoffes eine Dehnbarkeit von mindestens 30 %, vorzugsweise mindestens 40 %, besonders bevorzugt etwa 50 bis 100 % aufweist.

13. Material nach einem der vorhergehenden Ansprüche, **dadurch gekennzeichnet, daß** das Material in Richtung der Schußfäden (22, 24) des Gewebes in ungedehntem Zustand eine Reißfestigkeit von mindestens 10 N/cm, vorzugsweise mindestens 20 N/cm, besonders bevorzugt etwa 30 bis 50 N/cm aufweist.

14. Material nach einem der vorhergehenden Ansprüche, **dadurch gekennzeichnet, daß** das Gewebe in ungedehntem Zustand ein Flächengewicht von etwa 50 bis 250 g/cm², vorzugsweise etwa 100 bis 200 g/m² aufweist.

15. Material nach einem der vorhergehenden Ansprüche, **dadurch gekennzeichnet, daß** die Schuß- und/oder Kettfäden (12, 14, 22, 24, 30) des Gewebes Naturfasern aufweisen.

16. Material nach einem der vorhergehenden Ansprüche, **dadurch gekennzeichnet, daß** die Schuß- und/oder Kettfäden (12, 14, 22, 24, 30) des Gewebes Kunstfasern, wie etwa Polyamidfasern, Polyesterfasern, Polypropylenfasern oder dgl., aufweisen.

17. Material nach einem der Ansprüche 4 bis 11, **dadurch gekennzeichnet, daß** die Schuß- und/oder Kettfäden (12, 14, 22, 24, 30) zum Erhalt der Dehnbarkeit zumindest teilweise texturiert sind.

18. Material nach einem der vorhergehenden Ansprüche, **dadurch gekennzeichnet, daß** bei mindestens einem Paar sich kreuzender Kettfäden einer der sich kreuzenden Kettfäden (12) unterhalb der Schußfäden (22, 24) verläuft, der andere der sich kreuzenden Kettfäden (14) oberhalb der Schußfäden (22, 24) verläuft und der unterhalb der Schußfäden (22, 24) verlaufende Kettfaden (12) an den Kreuzungsstellen (16) oberhalb des oberhalb der Schußfäden (22, 24) verlaufenden Kettfadens (14) angeordnet ist.

19. Material nach einem der vorhergehenden Ansprüche, **dadurch gekennzeichnet, daß** mindestens zwei Schußfäden (22, 24) zwischen aufeinanderfolgenden Kreuzungsstellen (16) der Kettfäden (12, 14) und/oder aufeinanderfolgenden Übergängen mindestens eines zusätzlichen Kettfadens angeordnet sind.

20. Material nach Anspruch 19, **dadurch gekennzeichnet, daß** einer der zwischen aufeinanderfolgenden Kreuzungsstellen (16) und/oder aufeinanderfolgenden Übergängen angeordneten Schußfäden (22, 24) texturiert ist und ein anderer dieser Schußfäden (22, 24) aus einem hochfesten, vorzugsweise glatten Garn, insbesondere Polyestergarn, gebildet ist.

21. Material nach einem der vorhergehenden Ansprüche, **dadurch gekennzeichnet, daß** die Schußfäden (22, 24) in Form von Fadensegmenten gebildet sind, von denen mindestens zwei über ein am Rand des Gewebes angeordnetes Verbindungssegment miteinander verbunden sind.

22. Material nach Anspruch 21, **dadurch gekennzeichnet, daß** mindestens ein zwei Schußfäden miteinander verbindendes Verbindungssegment mindestens ein, vorzugsweise mindestens zwei vorhergehende Schußfäden umläuft.

23. Material nach einem der vorhergehenden Ansprüche, **dadurch gekennzeichnet, daß** das Gewebe in ungedehntem Zustand etwa 40 bis 80, vorzugsweise 66 Kettfäden und/oder etwa 80 bis 150, vorzugsweise 132 Schußfäden auf 10 cm der Gewebelänge bzw. -breite aufweist.

24. Material nach einem der vorhergehenden Ansprüche, **dadurch gekennzeichnet, daß** der Kunststoff mit einem Lösungsmittel, insbesondere Wasser, härtbar ist.

25. Material nach Anspruch 24, **dadurch gekennzeichnet, daß** der Kunststoff ein feuchtigkeitshärtendes Polyurethanprepolymer aufweist.

26. Material nach einem der vorhergehenden Ansprüche, **dadurch gekennzeichnet, daß** der Anteil des Kunststoffes an dem Material etwa 30 bis 70 Gew.-% beträgt.

27. Träger für ein Material zur Herstellung eines Stützverbandes, gebildet zumindest teilweise in Form eines zwei sich zwischen nebeneinander verlaufenden Schussfäden (22, 24) kreuzende Kettfäden (12, 14) aufweisenden Drehergewebes, **dadurch gekennzeichnet, dass** das Drehergewebe mindestens einen zwischen zwei Paaren (10) sich kreuzender Kettfäden (12, 14) angeordneten und keinen anderen Kettfaden kreuzenden zusätzlichen Kettfaden (30) aufweist.

28. Verfahren zum Herstellen eines Materials nach einem der Ansprüche 1 bis 26, **dadurch gekennzeichnet, daß** ein Träger nach Anspruch 27 mit einem härtbaren Kunststoff beschichtet und/oder imprägniert wird.

## Claims

1. A material for producing a support bandage comprising a support coated and/or impregnated with a curable plastic and at least partially in the form of a leno fabric having two warp yarns (12, 14) crossing between woof yarns (22, 24) extending next to one another, **characterised in that** the leno fabric has at least one additional warp yarn (30) disposed between two pairs (10) of crossing warp yarns (12, 14) and not crossing any other warp yarn.

2. The material according to Claim 1, **characterised in that** at least one additional warp yarn (30) has at least one second section extending on a side of the woof yarns (22, 24) facing away from a first section of this warp yarn (30), the cross-over between the first section and the second section preferably taking place between those woof yarns (22, 24) extending next to one another between which the warp yarns (12, 14) of at least one pair (10) of crossing warp yarns (12, 14) cross.

3. The material according to Claim 1 or 2, **characterised in that** of two additional warp yarns (30) disposed on opposite sides of a pair (10) of crossing warp yarns (30) one additional warp yarn (30) in the region of the woof yarns (22, 24) is disposed on the side of the woof yarns (22, 24) facing away from the other additional warp yarn (30).

4. The material according to any of Claims 1 to 3, **characterised in that** at least one additional warp yarn (30) is textured.

5. The material according to any of the preceding claims, **characterised in that** at least one warp yarn (12, 14) of a pair (10) of crossing warp yarns (12, 14) and/or at least one additional warp yarn (30) and/or at least one woof yarn (22, 24) is extendable, preferably elastically extendable, in particular at least partially made of an elastically extendable material, such as for example elastic polyurethane.

6. The material according to Claim 5, **characterised in that** the warp yarn (14) made at least partially of elastic material has a core made of elastic thread, such as for example polyurethane thread, wrapped preferably twice with a further thread, such as for example a polyester thread.

7. The material according to any of the preceding claims, **characterised in that** the leno fabric has at least one woof yarn (22) having a high-strength thread, in particular polyester thread.

8. The material according to any of the preceding claims, **characterised in that** the fabric has heat-shrinkable warp yarns and/or woof yarns.

9. The material according to any of the preceding claims, **characterised in that** the fabric has warp yarns and/or woof yarns made of an elastomer material.

10. The material according to any of the preceding claims, **characterised in that** the material has an extensibility of at least 20 %, preferably at least 30 %, particularly preferably approximately 65 to 95 % in the direction of the warp yarns (22, 24, 30) of the fabric before curing the plastic.

11. The material according to any of the preceding claims, **characterised in that** the material has a tensile strength of at least 10 N/cm, preferably at least 20 N/cm, particularly preferably approximately 30 to 60 N/cm in the direction of the warp yarns (22, 24, 30) of the fabric in the non-extended state.

12. The material according to any of the preceding claims, **characterised in that** the material has an extensibility of at least 30 %, preferably at least 40 %, particularly preferably approximately 50 to 100 % in the direction of the woof yarns (22, 24) of the fabric before curing the plastic.

13. The material according to any of the preceding claims, **characterised in that** the material has a tensile strength of at least 10 N/cm, preferably at least 20 N/cm, particularly preferably approximately 30 to 50 N/cm in the direction of the woof yarns (22, 24) of the fabric in the non-extended state.

14. The material according to any of the preceding claims, **characterised in that** the fabric has a mass per unit area of approximately 50 to 250 g/cm², preferably approximately 100 to 200 g/m² in the non-extended state.

15. The material according to any of the preceding claims, **characterised in that** the woof and/or warp yarns (12, 14, 22, 24, 30) of the fabric have natural fibres.

16. The material according to any of the preceding claims, **characterised in that** the woof and/or warp yarns (12, 14, 22, 24, 30) of the fabric have synthetic fibres, such as for example polyamide fibres, polyester fibres, polypropylene fibres or similar.

17. The material according to any of Claims 4 to 11, **characterised in that** the woof and/or warp yarns (12, 14, 22, 24, 30) are at least partially textured in order to retain the extensibility.

18. The material according to any of the preceding claims, **characterised in that** with at least one pair of crossing warp yarns, one of the crossing warp yarns (12) extends beneath the woof yarns (22, 24), the other of the crossing warp yarns (14) extends above the woof yarns (22, 24), and the warp yarn (12) extending beneath the woof yarns (22, 24) is disposed at the cross-over points (16) above the warp yarn (14) extending above the woof yarns (22, 24).

19. The material according to any of the preceding claims, **characterised in that** at least two woof yarns (22, 24) are disposed between consecutive cross-over points (16) of the warp yarns (12, 14) and/or consecutive cross-overs of at least one additional warp yarn.

20. The material according to Claim 19, **characterised in that** one of the woof yarns (22, 24) disposed between consecutive cross-over points (16) and/or consecutive cross-overs is textured, and another of these woof yarns (22, 24) is formed from a high-strength, preferably smooth thread, in particular polyester thread.

21. The material according to any of the preceding claims, **characterised in that** the woof yarns (22, 24) are in the form of yarn segments, at least two of which are connected to one another by means of a connection segment disposed on the edge of the fabric.

22. The material according to Claim 21, **characterised in that** at least one connection segment connecting two woof yarns to one another passes round at least one, preferably at least two, preceding woof yarns.

23. The material according to any of the preceding claims, **characterised in that** in the non-extended state the fabric has approximately 40 to 80, preferably 66 warp yarns and/or approximately 80 to 150, preferably 132 woof yarns over 10 cm of the fabric length or width.

24. The material according to any of the preceding claims, **characterised in that** the plastic can be cured with a solvent, in particular water.

25. The material according to Claim 24, **characterised in that** the plastic has a moisture-curing polyurethane prepolymer.

26. The material according to any of the preceding claims, **characterised in that** the plastic portion of the material is approximately 30 to 70 % by weight.

27. A support for a material for producing a support bandage, at least partially in the form of a leno fabric having two warp yarns (12, 14) crossing between woof yarns (22, 24) extending next to one another, **characterised in that** the leno fabric has at least one additional warp yarn (30) disposed between two pairs (10) of crossing warp yarns (12, 14) and not crossing any other warp yarn.

28. The method for producing a material according to any of Claims 1 to 26, **characterised in that** a support according to Claim 27 is coated and/or impregnated with a curable plastic.

## Revendications

1. Matériau destiné à produire une bande de maintien comportant un support qui est recouvert et/ou imprégné d'une matière plastique durcissable et qui se présente au moins partiellement sous la forme d'un tissu de gaze comportant deux fils de chaîne (12, 14) qui se croisent entre des fils de trame (22, 24) s'étendant côte à côte, **caractérisé en ce que** le tissu de gaze comporte au moins un fil de chaîne supplémentaire (30) qui est disposé entre deux paires (10) de fils de chaîne (12, 14) qui se croisent, et qui ne croise aucun autre fil de fil de chaîne.

2. Matériau selon la revendication 1, **caractérisé en ce qu'**au moins un fil de chaîne supplémentaire (30) présente une section, dite deuxième section, qui s'étend d'un côté des fils de trame (22, 24) détourné d'une première section dudit fil de chaîne (30), la transition entre la première section et la deuxième section se situant de préférence entre les fils de trame (22, 24) s'étendant côte à côte entre lesquels se croisent également les fils de chaîne (12, 14) d'au moins une paire (10) de fils de chaîne qui se croisent (12, 14).

3. Matériau selon la revendication 1 ou 2, **caractérisé en ce que**, sur deux fils de chaîne supplémentaires (30) situés sur des côtés mutuellement opposés d'une paire (10) de fils de chaîne (12, 14) qui se croisent, un fil de chaîne supplémentaire (30) à proximité des fils de trame (22, 24) se situe du côté des fils de trame (22, 24) qui est détourné de l'autre fil de chaîne supplémentaire (30).

4. Matériau selon l'une des revendications 1 à 3, **caractérisé en ce qu'**au moins un fil de chaîne supplémentaire (30) est texturisé.

5. Matériau selon l'une des revendications précédentes, **caractérisé en ce qu'**au moins un fil de chaîne (12, 14) d'une paire (10) de fils de chaîne qui se croisent (12, 14) et/ou au moins un fil de chaîne supplémentaire (30) et/ou au moins un fil de trame (22, 24) sont extensibles, de préférence de manière élastique, en étant composés notamment au moins partiellement d'un matériau élastiquement étirable comme par exemple le polyuréthane élastique.

6. Matériau selon la revendication 5, **caractérisé en ce que** le fil de chaîne (14) composé au moins partiellement d'un matériau élastique présente une âme de fil élastique, par exemple un fil de polyuréthane, portant un enroulement de préférence double d'un autre fil, tel qu'un fil de polyester.

7. Matériau selon l'une des revendications précédentes, **caractérisé en ce que** le tissu de gaze présente au moins un fil de trame (22) comportant un fil hautement résistant, notamment un fil de polyester.

8. Matériau selon l'une des revendications précédentes, **caractérisé en ce que** le tissu présente des fils de chaîne et/ou des fils de trame thermorétractables.

9. Matériau selon l'une des revendications précédentes, **caractérisé en ce que** le tissu présente des fils de chaîne et/ou des fils de trame composés d'un matériau élastomère.

10. Matériau selon l'une des revendications précédentes, **caractérisé en ce que** le matériau présente, dans la direction des fils de chaîne (22, 24, 30) du tissu, préalablement au durcissement de la matière plastique, une extensibilité d'au moins 20 %, de préférence d'au moins 30 %, et plus préférablement d'environ 65 à 95 %.

11. Matériau selon l'une des revendications précédentes, **caractérisé en ce que** le matériau présente, dans la direction des fils de chaîne (22, 24, 30) du tissu et à l'état non étiré, une résistance à la déchirure d'au moins 10 N/cm, de préférence d'au moins 20 N/cm, et plus préférablement d'environ 30 à 60 N/cm.

12. Matériau selon l'une des revendications précédentes, **caractérisé en ce que** le matériau présente, dans la direction des fils de trame (22, 24) du tissu, préalablement au durcissement de la matière plastique, une extensibilité d'au moins 30 %, de préférence d'au moins 40 %, et plus préférablement d'environ 50 à 100 %.

13. Matériau selon l'une des revendications précédentes, **caractérisé en ce que** le matériau présente, dans la direction des fils de trame (22, 24) du tissu et à l'état non étiré, une résistance à la déchirure d'au moins 10 N/cm, de préférence d'au moins 20 N/cm, et plus préférablement d'environ 30 à 50 N/cm.

14. Matériau selon l'une des revendications précédentes, **caractérisé en ce que** le tissu présente, à l'état non étiré, un poids surfacique d'environ 50 à 250 g/cm², de préférence d'environ 100 à 200 g/m².

15. Matériau selon l'une des revendications précédentes, **caractérisé en ce que** les fils de trame et/ou de chaîne (12, 14, 22, 24, 30) du tissu comportent des fibres naturelles.

16. Matériau selon l'une des revendications précédentes, **caractérisé en ce que** les fils de trame et/ou de chaîne (12, 14, 22, 24, 30) du tissu comportent des fibres artificielles telles que des fibres de polyamide, de polyester, de polypropylène ou similaires.

17. Matériau selon l'une des revendications 4 à 11, **caractérisé en ce que** les fils de trame et/ou de chaîne (12, 14, 22, 24, 30) sont au moins partiellement texturisés pour permettre leur extensibilité.

18. Matériau selon l'une des revendications précédentes, **caractérisé en ce que**, pour au moins une paire de fils de chaîne qui se croisent, l'un des fils de chaîne (12) qui se croisent passe par-dessous les fils de trame (22, 24), l'autre des fils de chaîne (14) qui se croisent passe pardessus les fils de trame (22, 24) et le fil de chaîne (12) passant sous les fils de trame (22, 24) est situé, au niveau des points de croisement (16), au-dessus du fil de chaîne (14) passant au-dessus des fils de trame (22, 24).

19. Matériau selon l'une des revendications précédentes, **caractérisé en ce qu'**au moins deux fils de trame (22, 24) sont situés entre des points de croisement (16) consécutifs des fils de chaîne (12, 14) et/ou des transitions consécutives d'au moins un fil de chaîne supplémentaire.

20. Matériau selon la revendication 19, **caractérisé en ce qu'**un des fils de trame (22, 24) situés entre des points de croisement (16) consécutifs et/ou des transitions consécutives est texturisé et un autre de ces fils de trame (22, 24) consiste en un fil hautement résistant, de préférence lisse, notamment un fil de polyester.

21. Matériau selon l'une des revendications précédentes, **caractérisé en ce que** les fils de trame (22, 24) se présentent sous la forme de segments de fil parmi lesquels au moins deux sont reliés l'un à l'autre par le biais d'un segment de liaison situé sur le bord du tissu.

22. Matériau selon la revendication 21, **caractérisé en ce qu'**au moins un segment de liaison reliant l'un à l'autre deux fils de trame est enroulé autour d'au moins un, de préférence au moins deux, fils de trame précédents.

23. Matériau selon l'une des revendications précédentes, **caractérisé en ce que** le tissu présente, à l'état non étiré, d'environ 40 à 80, de préférence 66 fils de chaîne et/ou d'environ 80 à 150, de préférence 132 fils de trame pour une longueur, voire une largeur, de tissu de 10 cm.

24. Matériau selon l'une des revendications précédentes, **caractérisé en ce que** la matière plastique est durcissable sous l'effet d'un solvant, notamment de l'eau.

25. Matériau selon la revendication 24, **caractérisé en ce que** la matière plastique comporte un prépolymère polyuréthane durcissable à l'humidité.

26. Matériau selon l'une des revendications précédentes, **caractérisé en ce que** la proportion de matière plastique par rapport au matériau est d'environ 30 à 70 % en poids.

27. Support pour un matériau destiné à produire une bande de maintien, se présentant au moins partiellement sous la forme d'un tissu de gaze comportant deux fils de chaîne (12, 14) qui se croisent entre des fils de trame (22, 24) s'étendant côte à côte, **caractérisé en ce que** le tissu de gaze comporte au moins un fil de chaîne supplémentaire (30) qui est disposé entre deux paires (10) de fils de chaîne (12, 14) qui se croisent, et qui ne croise aucun autre fil de chaîne.

28. Procédé de production d'un matériau selon l'une des revendications 1 à 26, **caractérisé en ce qu'**un support selon la revendication 27 est recouvert et/ou imprégné d'une matière plastique durcissable.
